(19) Europäisches Patentamt
European Patent Office
Office européen des brevets

(11) Veröffentlichungsnummer: **0 369 266 B1**

(12) **EUROPÄISCHE PATENTSCHRIFT**

(45) Veröffentlichungstag der Patentschrift: **08.11.95**

(51) Int. Cl.⁶: **A61B 17/58**, F16B 35/04

(21) Anmeldenummer: **89120436.4**

(22) Anmeldetag: **04.11.89**

(54) **Knochenschraube.**

(30) Priorität: **17.11.88 CH 4263/88**

(43) Veröffentlichungstag der Anmeldung:
**23.05.90 Patentblatt 90/21**

(45) Bekanntmachung des Hinweises auf die
Patenterteilung:
**08.11.95 Patentblatt 95/45**

(84) Benannte Vertragsstaaten:
**AT DE FR GB**

(56) Entgegenhaltungen:
**EP-A- 0 230 856**
**DE-U- 7 625 614**
**FR-A- 2 082 457**
**US-A- 4 463 753**

(73) Patentinhaber: **Synthes AG, Chur**
**Grabenstrasse 15**
**CH-7002 Chur (CH)**

(72) Erfinder: **Perren, Stephan M.**
**Dischmastrasse 22**
**CH-7260 Davos-Dorf (CH)**
Erfinder: **Frigg, Robert**
**Promenade 5**
**CH-7270 Davos-Dorf (CH)**
Erfinder: **Gisin, Paul**
**Brestenbergweg 7**
**CH-4437 Waldenburg (CH)**
Erfinder: **Mathys, Robert**
**Chrüzliacher 11**
**CH-2544 Bettlach (CH)**

(74) Vertreter: **Lusuardi, Werther Giovanni, Dr.**
**Dr. Lusuardi AG,**
**Kreuzbühlstrasse 8**
**CH-8008 Zürich (CH)**

Rank Xerox (UK) Business Services
(3.10/3.09/3.3.3)

## Beschreibung

Die Erfindung bezieht sich auf eine Knochenschraube mit einem Schraubenkopf (1), einem glatten Schaftteil (2) und einem Gewindeteil (3) mit dem Innendurchmesser $d_1$ und dem Aussendurchmesser $d_2$. Eine solche Schraube ist aus dem Dokument DE-U-7 625 614 bekannt.

Die Verwendung solcher Schrauben gemäss dem Oberbegriff des Anspruchs 1, auch Schanzsche Schrauben genannt, ist mit einer Reihe wesentlicher Nachteile behaftet.
So wird bei jeder zusätzlich auftretenden, funktionellen Belastung der Schraube in radialer Richtung auf der einen Seite der Pressitz verstärkt, auf der entgegengesetzten Seite jedoch - wegen der fehlenden Vorspannung - gleichzeitig aufgehoben, was zu einer entsprechenden Lockerung und nachfolgenden Knochenresorption führen kann [siehe: S.M.Perren, R.Ganz und A.Rüter, Med. Orthop. Technik, Heft 1/75 95.Jahrg.(1975), pag. 6-10]. Zudem sind bei Schanzschen Schrauben heutiger Bauart zwei Bohrvorgänge notwendig: Ein Bohrvorgang mit dem Innendurchmesser $d_1$ und ein Bohrvorgang mit dem Aussendurchmesser $d_2$ des Gewindes. Versucht man den operativen Vorgang zu vereinfachen durch Ausführung einer einzigen, unterdimensionierten Bohrung, so erhält man Mikro- oder Makrofrakturen im Knochenmaterial. Bei der heutigen Konstruktion solcher Schanzschen Schrauben ist nämlich der Übergang zwischen Schaft- und Gewindeteil, welche beide den gleichen Aussendurchmesser aufweisen, als Hohlkehle ausgebildet, die entweder an ihrer Vorderkante das Knochenmaterial stanzt oder an ihrer Hinterkante schneidet. Es findet somit nicht allein ein radiales Auseinanderpressen des Knochenmaterials in der Bohrung statt, das idealerweise gefordert werden muss.

Hier will die Erfindung Abhilfe schaffen. Der Erfindung liegt die Aufgabe zugrunde, eine Knochenschraube der eingangs erwähnten Art zu schaffen, welche mit nur einem Bohrvorgang unter radialer Kompression des aufnehmenden Knochengewebes fest in eine unterdimensionierte Bohrung, ohne eine Knochenverletzung zu erzeugen, eingeschraubt werden kann, und welche auch unter zusätzlichen, funktionellen Belastungen den radialen Pressitz über den gesamten Umfang beibehält.

Die Erfindung löst die gestellte Aufgabe mit einer Knochenschraube, welche die Merkmale des Anspruchs 1 aufweist.

Die durch die Erfindung erreichten Vorteile sind im wesentlichen darin zu sehen, dass dank der erfindungsgemässen Knochenschraube eine erwünschte radiale Vorspannung in der Knochenbohrung erzeugt wird, welche auch bei zusätzlichen, funktionellen Belastungen der Knochenschraube mindestens teilweise aufrechterhalten bleibt, so dass keine Lockerungen und dadurch bedingte Resorptionen des Knochengewebes auftreten; im weiteren muss nur noch ein einziger gängiger Bohrer verwendet werden (z.B. mit einem Durchmesser von 3,5 mm) um den Schaft der Knochenschraube fest, aber ohne Knochenverletzung in die Bohrung eindrehen zu können.

Da die Bruchdehnung des Knochens bei etwa 2 - 3 % liegt sollten die Dimensionen des Übergangsteiles der erfindungsgemässen Knochenschraube derart gewählt werden, dass das Verhältnis $D_1$-$D_2$/$D_2$ zwischen 0,001 und 0,040, vorzugsweise zwischen 0,010 und 0,030 liegt. Damit kann eine optimale Pressung des Knochenmaterials erreicht werden.

Der Durchmesser $D_2$ entspricht mit Vorteil dem Aussendurchmesser $d_2$ des Schraubengewindes um eine genügende Pressung des Knochenmaterials durch den glatten Schaftteil zu garantieren.

Bei einer bevorzugten Ausführungsform ist der Übergangsteil als selbstschneidender Gewindeschaftübergang, vorzugsweise mit einer oder mehreren radial über den Umfang verteilten Hohlkehlen ausgebildet, der kontinuierlich vom Aussendurchmesser $D_1$ zum Durchmesser $D_2$, vorzugsweise in Form eines Konus, abnimmt.

Um den Vortrieb der Schraube beim Eindrehen in den Knochen auch vor Erreichen der hinteren Kortikaliswand zu gewährleisten, kann das Übergangsteil mit einem leichten Gewinde (geringer Gangtiefe) versehen werden.

Eine weitere zusätzliche Verbesserungen kann dadurch erreicht werden, dass zwischen Gewindeteil und Übergangsteil zusätzlich ein, vorzugsweise konisch ausgebildeter, Frästeil vorgesehen wird.

Das Gewinde der Knochenschraube ist zweckmässigerweise derart dimensioniert, dass das Verhältnis zwischen Innendurchmesser $d_1$ und Aussendurchmesser $d_2$ im Bereich zwischen 0,80 und 0,89, vorzugsweise zwischen 0,83 und 0,86 liegt.

Eine weitere Verbesserung kann dadurch erreicht werden, dass am freien Ende des Gewindeteils eine selbstaufweitende Schraubenspitze, vorzugsweise eine Trokarspitze, die auch korkzieherartig ausgebildet sein kann, angebracht ist.
Um zu verhindern, dass gleichzeitig das Gewindeteil in der hinteren Kortikalis und das Übergangsteil in der vorderen Kortikalis zur Wirkung kommen, wodurch man die Kontrolle beim Eindrehen der Knochenschraube verlieren würde, ist es vorteilhaft den Übergangsteil in einem Abstand von 11 - 15 mm, vorzugsweise von 12 - 14 mm vom freien Ende des Gewindeteils anzubringen.

Ein Ausführungsbeispiel der Erfindung, welches zugleich das Funktionsprinzip erläutert, ist in der Zeichnung dargestellt und wird im folgenden näher beschrieben.

Fig. 1 stellt einen teilweisen Schnitt durch die erfindungsgemässe Knochenschraube dar;
Fig. 2 stellt einen teilweisen Schnitt durch die erfindungsgemässe Knochenschraube mit einem zusätzlichen Frästeil dar; und
Fig. 3 stellt einen teilweisen Schnitt durch die erfindungsgemässe Knochenschraube dar, bei welcher das Übergangsteil ein Gewinde aufweist.

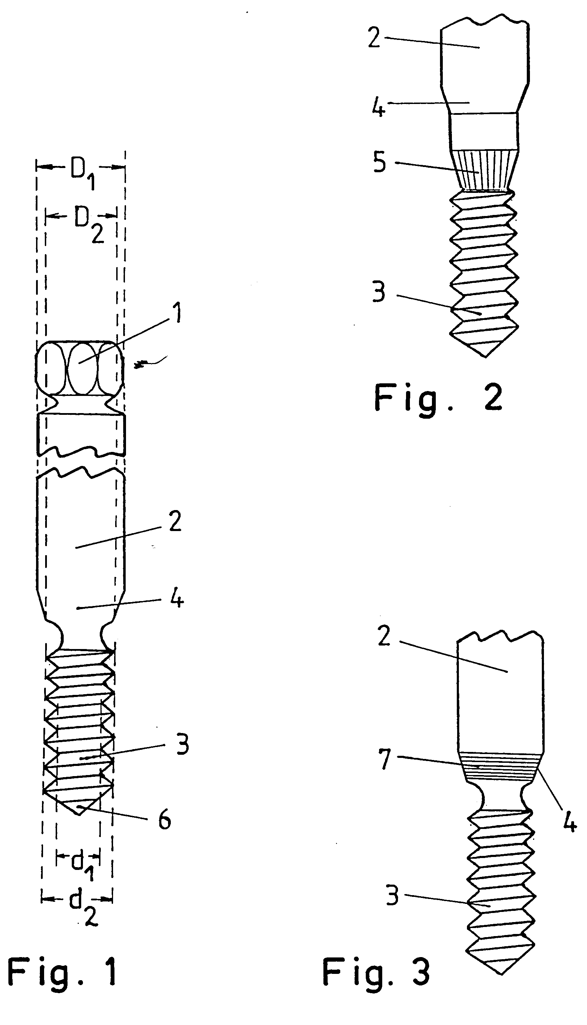

Die in Fig. 1 dargestellte Knochenschraube besteht im wesentlichen aus einem Schraubenkopf 1, einem glatten Schaftteil 2 mit einem Durchmesser $D_1$ von 4,5 mm und einem Gewindeteil 3 mit einem Innendurchmesser $d_1$ von 3,8 mm und einem Aussendurchmesser $d_2$ von 4,4 mm. Der Übergangsteil 4 zwischen glattem Schaftteil 2 und Gewindeteil 3 ist als Konus ausgebildet, der kontinuierlich vom Durchmesser $D_2$ zum Durchmesser $D_1$ zunimmt, und ist als selbstschneidender Gewindeschaftübergang ausgebildet. Das freie Ende des Gewindeteils 3 ist als selbstaufweitende Schraubenspitze (Trokarspitze) 6 ausgebildet, wobei der Abstand zwischen dem Übergangsteil 4 und dem freien Ende des Gewindeteils 3 auf etwa 13 mm bemessen ist.

Die in Fig. 2 dargestellte erfindungsgemässe Knochenschraube weist zwischen Gewindeteil 3 und Übergangsteil 4 zusätzlich einen Frästeil 5 vor. Der Übergangsteil 4 ist bei dieser Ausführungsform als Schulter ausgebildet.

Die in Fig. 3 dargestellte erfindungsgemässe Knochenschraube besitzt am Übergangsteil 4 zusätzlich ein leichtes Gewinde 7.

Die Verwendung der erfindungsgemässen Knochenschraube erfolgt durch Vorbohren eines Loches in den Knochen mit einem gängigen Bohrer von 3,5 mm Durchmesser und Eindrehen der Knochenschraube mit den üblichen Instrumenten.

**Patentansprüche**

1. Knochenschraube mit einem Schraubenkopf (1), einem glatten Schaftteil (2) und einem Gewindeteil (3) mit dem Innendurchmesser $d_1$ und dem Aussendurchmesser $d_2$, wobei zwischen dem glatten Schaftteil (2) und dem Gewindeteil (3) ein Übergangsteil (4) vorgesehen ist, der sich vom glatten Schaftteil (2) mit dem Durchmesser $D_1$ zum Gewindeteil (3) hin auf den Durchmesser $D_2$ verjüngt; dadurch gekennzeichnet dass
A) das Verhältnis $(D_1-D_2)/D_2$ zwischen 0,001 und 0,040 liegt; und
B) der Durchmesser $D_2$ des glatten Schaftteils (2) in Relation zum Aussendurchmesser $d_2$ des Gewindeteils (3) derart gewählt ist, dass eine Vorspannung in der Knochenbohrung erzeugt wird.

2. Knochenschraube nach Anspruch 1, dadurch gekennzeichnet, dass das Verhältnis $(D_1-D_2)/D_2$ zwischen 0,010 und 0,030 liegt.

3. Knochenschraube nach Anspruch 1 oder 2, dadurch gekennzeichnet, daß die Relation zwischen Aussendurchmesser $d_2$ und Durchmesser $D_2$ der Bedingung $d_2 \leq D_2$ gehorcht.

4. Knochenschraube nach Anspruch 1, 2 oder 3, dadurch gekennzeichnet, dass der Durchmesser $D_2$ dem Aussendurchmesser $d_2$ des Gewindes (3) entspricht.

5. Knochenschraube nach einem der Ansprüche 1 bis 4, dadurch gekennzeichnet, dass der Übergangsteil (4) als selbstschneidender Gewindeschaftübergang, vorzugsweise mit einer oder mehreren im wesentlichen in Längsrichtung der Knochenschraube verlaufenden Hohlkehlen ausgebildet ist.

6. Knochenschraube nach einem der Ansprüche 1 bis 5, dadurch gekennzeichnet, dass der Übergangsteil (4) kontinuierlich vom Aussendurchmesser $D_1$ zum Durchmesser $D_2$, vorzugsweise in Form eines Konus, abnimmt.

7. Knochenschraube nach einem der Ansprüche 1 bis 6, dadurch gekennzeichnet, dass der Übergangsteil (4) mit einem leichten Gewinde (7) versehen ist.

8. Knochenschraube nach einem der Ansprüche 1 bis 7, dadurch gekennzeichnet, dass zwischen Gewindeteil (3) und Übergangsteil (4) zusätzlich ein, vorzugsweise konisch ausgebildeter, Frästeil (5) vorgesehen ist.

9. Knochenschraube nach einem der Ansprüche 1 bis 8, dadurch gekennzeichnet, dass das Verhältnis zwischen Innendurchmesser $d_1$ und Aussendurchmesser $d_2$ im Bereich zwischen 0,80 und 0,89, vorzugsweise zwischen 0,83 und 0,86 liegt.

10. Knochenschraube nach einem der Ansprüche 1 bis 9, dadurch gekennzeichnet, dass am freien Ende des Gewindeteils (3) eine selbstaufweitende Schraubenspitze (6), vorzugsweise eine Trokarspitze angebracht ist.

11. Knochenschraube nach einem der Ansprüche 1 bis 10, dadurch gekennzeichnet, dass der Übergangsteil (4) in einem Abstand von 11 - 15 mm, vorzugsweise von 12 - 14 mm vom freien Ende des Gewindeteils (3) angebracht ist.

**12.** Knochenschraube nach Anspruch 10 oder 11, dadurch gekennzeichnet, dass die Schraubenspitze (6) korkzieherartig ausgebildet ist.

## Claims

**1.** Bone screw with a screw head (1), a smooth shaft segment (2), and a threaded segment (3) with interior diameter $d_1$ and exterior diameter $d_2$, whereby a transition segment (4) is provided between the smooth shaft segment (2) and the threaded segment (3), said transition segment (4) tapering from smooth shaft segment (2) with diameter $D_1$ to threaded segment (3) with diameter $D_2$, characterized in that the ratio $(D_1-D_2)/D_2$ is between 0,001 and 0,040; and that the diameter $D_2$ of the smooth shaft segment (2) is selected in such a way in relation to the exterior diameter $d_2$ of the threaded segment (3) that a prestress is generated in the bone hole.

**2.** Bone screw according to claim 1, characterized in that the ratio $(D_1-D_2)/D_2$ is between 0,010 and 0,030.

**3.** Bone screw according to claim 1 or 2, characterized in that the relation between exterior diameter $d_2$ and diameter $D_2$ is governed by the condition $d_2 \leq D_2$.

**4.** Bone screw according to claim 1, 2 or 3, characterized in that the diameter $D_2$ corresponds to the exterior diameter $d_2$ of the threaded segment (3).

**5.** Bone screw according to one of claims 1 to 4, characterized in that the transition segment (4) is constructed as a self-cutting thread shaft transition, preferably with one or more channels running essentially in the longitudinal direction of the bone screw.

**6.** Bone screw according to one of claims 1 to 5, characterized in that the transition segment (4) narrows continuously from exterior diameter $D_1$ to diameter $D_2$, preferably in the form of a cone.

**7.** Bone screw according to one of claims 1 to 6, characterized in that the transition segment (4) has a shallow threading (7).

**8.** Bone screw according to one of claims 1 to 7, characterized in that in addition a milling segment (5), preferably conical, is positioned between the threaded segment (3) and the transition segment (4).

**9.** Bone screw according to one of claims 1 to 8, characterized in that the ratio between interior diameter $d_1$ and exterior diameter $d_2$ ranges between 0,80 and 0,89, and preferably between 0,83 and 0,86.

**10.** Bonce screw according to one of claims 1 to 9, characterized in that an automatically expanding screw tip (6), preferably a Trokar tip, is positioned at the free end of the threaded segment (3).

**11.** Bone screw according to one of claims 1 to 10, characterized in that the transition segment (4) is positioned at a distance of from 11 to 15 mm, preferably from 12 to 14 mm, from the free end of the threaded segment (3).

**12.** Bone screw according to claim 10 or 11, characterized in that the screw tip (6) is constructed like a corkscrew.

## Revendications

**1.** Vis osseuse comportant une tête de vis (1), une partie de tige lisse (2) et une partie filetée (3) d'un diamètre interne $d_1$ et d'un diamètre externe $d_2$, où entre la partie de tige lisse (2) et la partie filetée (3) est prévue une partie de transition (4) dont le diamètre se rétrécit depuis la partie de tige lisse (2) de diamètre $D_1$ jusqu'à la partie filetée (3) de diamètre $D_2$; caractérisée en ce que le rapport $(D_1-D_2)/D_2$ vaut entre 0,001 et 0,040; et en ce que le diamètre $D_2$ de la partie de tige lisse (2) est choisi en relation avec le diamètre externe $d_2$ de la partie filetée (3), de telle sorte que soit créée une précontrainte dans l'alésage de l'os.

**2.** Vis osseuse selon la revendication 1, caractérisée en ce que le rapport $(D_1-D_2)/D_2$ est situé entre 0,010 et 0,030.

**3.** Vis osseuse selon la revendication 1 ou 2, caractérisée en ce que la relation entre le diamètre externe $d_2$ et le diamètre $D_2$ vérifie la condition $d_2 \leq D_2$.

**4.** Vis osseuse selon la revendication 1, 2 ou 3, caractérisée en ce que le diamètre $D_2$ correspond au diamètre externe $d_2$ du filet (3).

**5.** Vis osseuse selon l'une quelconque des revendications 1 à 4, caractérisée en ce que la partie de transition (4) est configurée comme transition filet-tige auto-taraudeuse, de préférence avec une ou plusieurs gorges creuses s'étendant essentiellement dans la direction longitudinale de la vis osseuse.

6. Vis osseuse selon l'une quelconque des revendications 1 à 5, caractérisée en ce que la partie de transition (4) diminue de manière continue depuis le diamètre externe $D_1$ jusqu'au diamètre $D_2$, de préférence sous la forme d'un cône.

7. Vis osseuse selon l'une quelconque des revendications 1 à 6, caractérisée en ce que la partie de transition (4) est pourvue d'un léger filet (7).

8. Vis osseuse selon l'une quelconque des revendications 1 à 7, caractérisée en ce qu'entre la partie filetée (3) et la partie de transition (4) est de plus prévue une partie de fraisage (5) configurée de préférence en forme de cône.

9. Vis osseuse selon l'une quelconque des revendications 1 à 8, caractérisée en ce que le rapport entre le diamètre interne $d_1$ et le diamètre externe $d_2$ est situé dans la plage de 0,80 à 0,89, et de préférence entre 0,83 et 0,86.

10. Vis osseuse selon l'une quelconque des revendications 1 à 9, caractérisée en ce qu'à l'extrémité libre de la partie filetée (3) est installée une pointe de vis auto-évasante (6), de préférence une pointe Trokar.

11. Vis osseuse selon l'une quelconque des revendications 1 à 10, caractérisée en ce que la partie de transition (4) est installée à une distance de 11-15 mm, de préférence de 12-14 mm de l'extrémité libre de la partie filetée (3).

12. Vis osseuse selon la revendication 10 ou 11, caractérisée en ce que la pointe de vis (6) est configurée en forme de tire-bouchon.

Fig. 1

Fig. 2

Fig. 3